# EUROPEAN PATENT APPLICATION

(11) **EP 4 494 639 A1**
(43) Date of publication of application: **22.01.2025**
(21) Application number: 23771007.4
(22) Date of filing: 03.03.2023
(51) Int. Cl.: A61K 31/385, A61K 31/713, A61P 21/00, A23L 33/10, A23L 33/13

(54) **COMPOSITION COMPRISING ALOX5 INHIBITOR FOR PREVENTING OR TREATING SARCOPENIA**

(30) Priority: 16.03.2022 KR 20220032653
(71) Applicant: Pluto Inc., Seongnam-si Gyeonggi-do 13453 (KR)
(72) Inventor: DARREN, Williams, Gwangju 61005 (KR); JUNG, Da Woon, Gwangju 61005 (KR); KIM, Hyun Jun, Gwangju 61005 (KR)
(74) Representative: Høiberg P/S
(86) International application number: PCT/KR2023/002932
(87) International publication number: WO 2023/177128

(57) **Abstract**

The present invention relates to a composition comprising an Alox5 inhibitor for preventing or treating sarcopenia, and comprises a material that targets Alox5 so as to inhibit the expression or activation thereof, and thus improves muscular performance, increases muscle mass and cross-sectional areas of muscle fibers, and reduces the expression of myoatrophy regulators, thereby effectively preventing, alleviating and treating sarcopenia.

## Description

### [Technical Field]

The present invention relates to a composition for preventing or treating sarcopenia comprising an Alox5 inhibitor.

### [Background Art]

The concept of sarcopenia began in 1989 when Irwin Rosenberg introduced the term 'sarcopenia'. Looking at its origins in Greek, it is a combination of the words "sarco" meaning muscle and "penia" meaning decreased. Sarcopenia is associated with aging and refers to a decrease in muscle strength due to a decrease in muscle mass. Here, "muscle" refers to skeletal muscle and has nothing to do with smooth muscle. In other words, sarcopenia refers to a loss of skeletal muscle mass mainly distributed in the extremities, and it is distinguished from cachexia, a state of significant muscle loss that occurs in the terminal stages of malignant tumors, muscle wasting due to acute diseases such as the flu, or primary muscle disease.

Recently, as the age group of 65 years or older has rapidly increased, the prevalence of osteoporosis and sarcopenia is also rapidly increasing. It is estimated that a gradual decrease in muscle mass occurs after the age of 40, with an 8% decrease every 10 years until the 70s. After that, it is known that a more rapid decrease occurs, up to 15% every 10 years. there is. Many follow-up studies have revealed that the physiological changes that occur in the elderly are diverse, and that muscle mass and bone density generally decrease simultaneously with age.

Meanwhile, various studies are being conducted on ways to efficiently control sarcopenia. For example, growth hormone (GH) has been developed to increase muscle mass, but it is very expensive and causes some undesirable side effects, such as shortening the average age. Mitochondrial stabilizing drugs also showed no effect on sarcopenia. In addition, as it is a very inappropriate method for patients or patients lying in a hospital bed, the development of drugs and technologies to treat sarcopenia that can induce muscle regeneration and differentiation is becoming a major task. Although research is being conducted on this, it is still insufficient.

Accordingly, in a society where aging continues, the demand for pharmaceutical compositions and functional health foods that are effective in sarcopenia is expected to continue to increase, and research on new drugs that can be effective in sarcopenia is needed.

### [Disclosure]

### [Technical Problem]

The technical problem to be achieved by the present invention is to provide a pharmaceutical composition for preventing or treating sarcopenia.

In addition, the technical problem to be achieved by the present invention is to provide a functional health food for preventing or improving sarcopenia.

The technical problem to be achieved by the present invention is not limited to the technical problem mentioned above, and other technical problems not mentioned can be clearly understood by those skilled in the art from the description below.

### [Technical Solution]

In order to achieve the above technical object, an embodiment of the present invention provides a pharmaceutical composition for preventing or treating sarcopenia comprising an Alox5 (Arachidonate 5-Lipoxygenase) inhibitor.

In an embodiment of the present invention, the Alox5 inhibitor may be a nucleic acid, antibody, aptamer, peptide, protein, compound, or natural product.

In an embodiment of the present invention, the Alox5 inhibitor is selected from the group consisting of Malotilate, Wedelolactone, Zileuton, Nordihydroguaiaretic acid, Psoralidin, Docebenone, Licofelone, Lonapalene, Enazadrem, Cirsiliol, Picrinine, Atreleuton and mixtures thereof.

In an embodiment of the present invention, the prevention or treatment of sarcopenia may be achieved by reducing the production of leukotriene B4 (LTB4).

In an embodiment of the present invention, the prevention or treatment of sarcopenia may be achieved by reduced expression of Atrogin-1 or MuRF-1; or increased expression of one or more genes selected from Efna5 (Ephrin A5), Fut4 (Fucosyltransferase 4), Igf1 (Insulin-like growth factor 1), Sod3 (Superoxide dismutase 3), and Plk 1 (Polo like kinase1).

In an embodiment of the present invention, the sarcopenia may be due to aging.

In order to achieve the above technical object, another embodiment of the present invention provides a functional health food for preventing or improving sarcopenia comprising an Alox5 (Arachidonate 5-Lipoxygenase) inhibitor.

In an embodiment of the present invention, the Alox5 inhibitor may be a nucleic acid, antibody, aptamer, peptide, protein, compound, or natural product.

In an embodiment of the present invention, the Alox5 inhibitor is selected from the group consisting of Malotilate, Wedelolactone, Zileuton, Nordihydroguaiaretic acid, Psoralidin, Docebenone, Licofelone, Lonapalene, Enazadrem, Cirsiliol, Picrinine, Atreleuton and mixtures thereof.

In an embodiment of the present invention, the prevention or treatment of sarcopenia may be achieved by reducing the production of leukotriene B4 (LTB4).

In an embodiment of the present invention, the prevention or treatment of sarcopenia may be achieved by reduced expression of Atrogin-1 or MuRF-1; or increased expression of one or more genes selected from Efna5 (Ephrin A5), Fut4 (Fucosyltransferase 4), Igf1 (Insulin-like growth factor 1), Sod3 (Superoxide dismutase 3), and Plk 1 (Polo like kinase1).

In an embodiment of the present invention, the sarcopenia may be due to aging.

### [Advantageous Effects]

The present invention relates to a composition for preventing or treating sarcopenia comprising an Alox5 inhibitor, that is the composition comprises the substance that targets Alox5 and inhibits its expression or activity, thereby improving muscle performance and increasing muscle mass and muscle fiber cross-sectional area. By reducing the expression of muscle atrophy regulators, sarcopenia can be effectively prevented, improved, and treated.

The effects of the present invention are not limited to the effects described above, and should be understood to include all effects that can be inferred from the configuration of the invention described in the description or claims of the present invention.

### [Brief Description of Drawings]

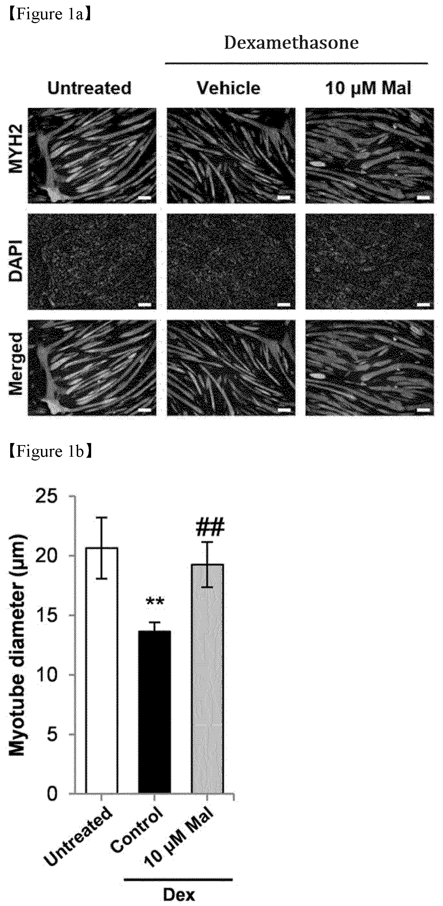

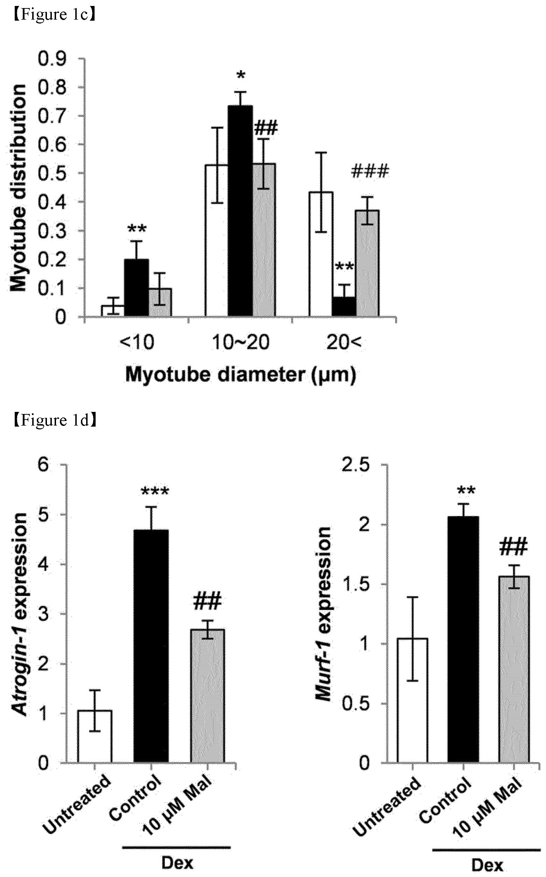

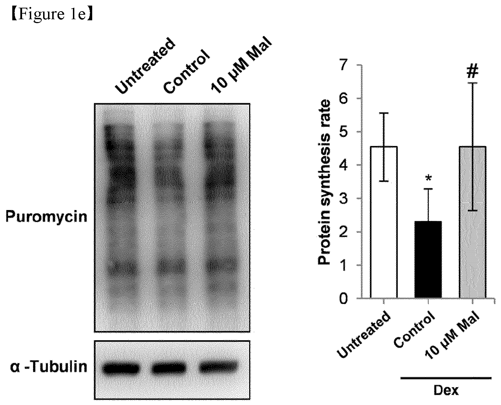

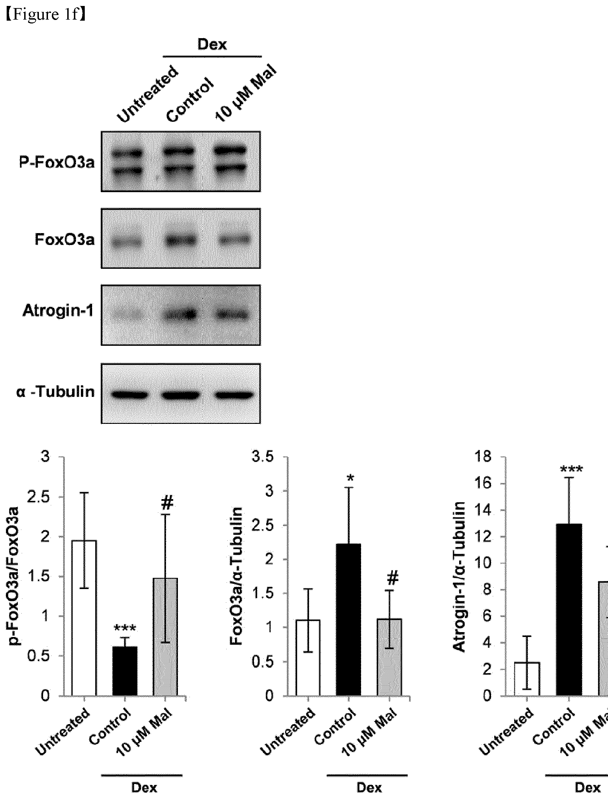

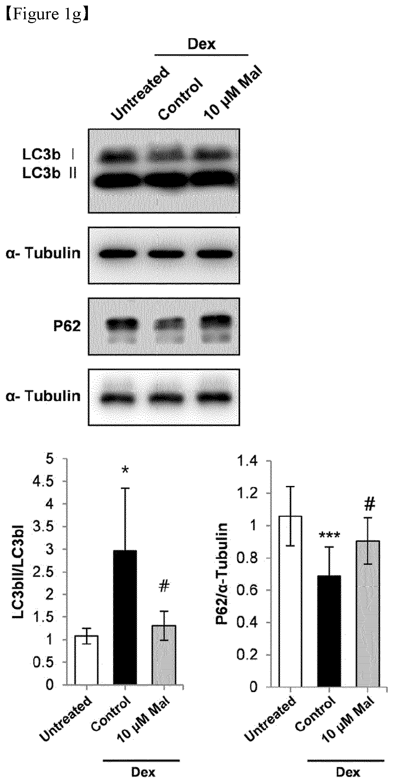

Figures 1a to 1g confirm the effect of inhibiting atrophy by malotilate treatment in Dex-treated mouse myotubes. Figure 1a shows C2C12 muscle fibers stained with myosin heavy chain immunochemical staining. Figure 1b shows the average muscle fiber diameter. Figure 1c shows the distribution of muscle fiber diameter compared to the total number of muscle fibers. Figure 1d analyzes the expression levels of Atrogin-1 and Murf-1 at the mRNA level through qPCR analysis. Figure 1e compares and analyzes protein synthesis rates through SUnSET assay. Figure 1f shows the degree of phosphorylation of FoxO3a and the expression of FoxO3a and Atrogin-1 through Western blot, and the expression levels of the corresponding genes were quantified through the expression level of α-Tubulin. Figure 1g shows the expression levels of LC3b I, LC3b II, and p62 through Western blot, and the expression levels of the corresponding genes were quantified through the expression level of α-Tubulin (*=p<0.05, **=p<0.01, and ***=p<0.001 mean statistical significance compared to the untreated group, and #=p<0.05, ##=p<0.01, and ###=p<0.001 mean statistical significance compared to the Dex group).

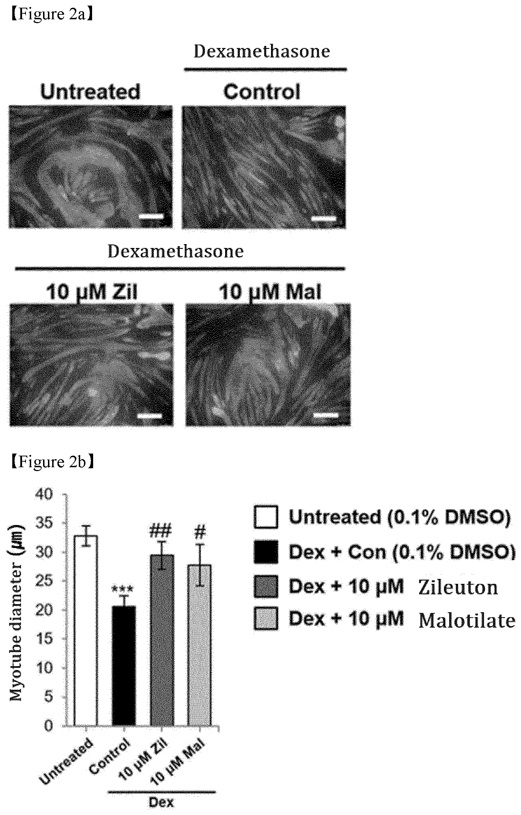

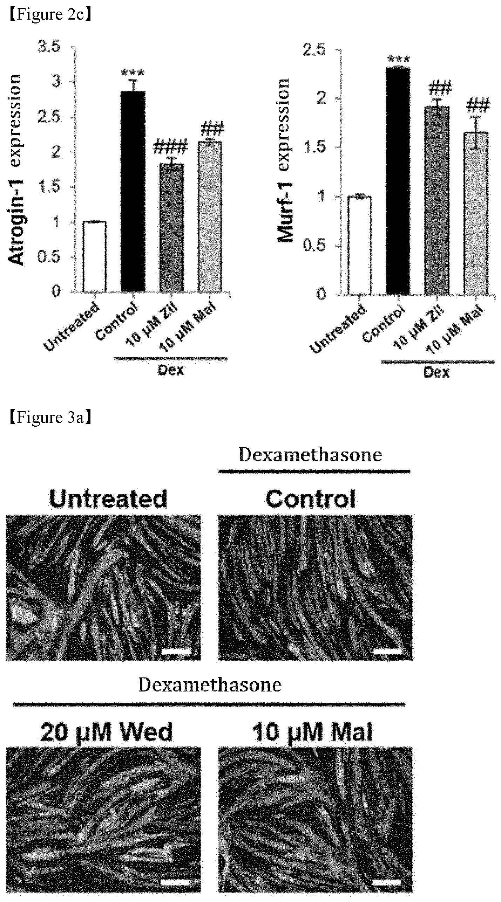

Figures 2a to 2c confirm the effect of inhibiting atrophy by zileuton and malotilate treatment in the myotubes of mice treated with the glucocorticoid. Figure 2a shows representative images of C2C12 muscle fibers stained with myosin heavy chain immunochemical staining (Representative images about myosin heavy chain2 staining). The cells were cultured as follows: (1) Cultured in differentiation medium for 120 h (Untreated); (2) Cultured in differentiation medium for 96 h, and then additionally treated with DM and 10 µM Dex for 24 h (Dex); and (3) Cultured in differentiation medium for 96 h, and then additionally treated with DM, 10 µM Dex, and 10 µM Zileuton for 24 h (Mal) (size bar = 150 µm). Figure 2b shows the average myotube diameter measurement. Figure 2c shows the expression level of Atrogin-1 and Murf-1 at the mRNA level through qPCR analysis (*=p<0.05, **=p<0.01, and ***=p<0.001 mean statistical significance compared to the untreated group, and #=p<0.05, ##=p<0.01, and ###=p<0.001 mean statistical significance compared to the Dex-treated control group).

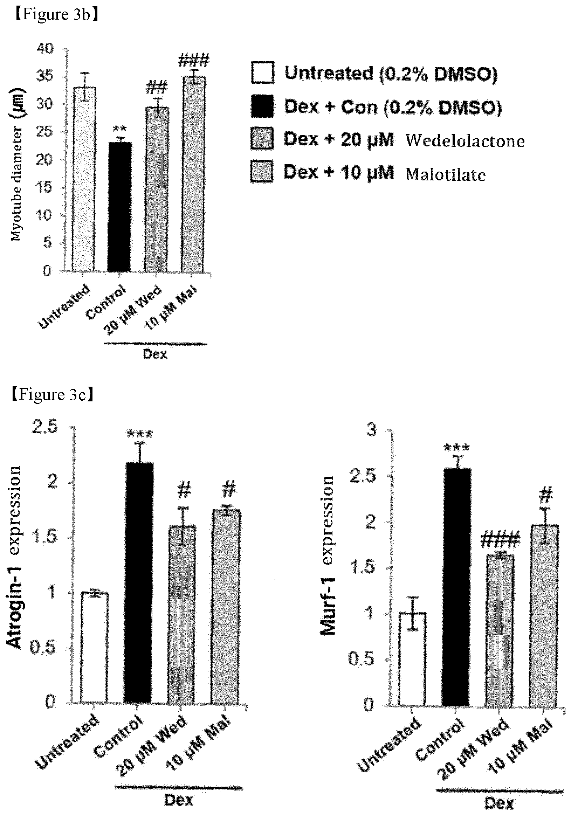

Figures 3a to 3c confirm the effect of inhibiting atrophy by wedelolactone and malotilate treatment in the myotubes of mice treated with the glucocorticoid. Figure 3a shows representative images of C2C12 myofibers stained with myosin heavy chain immunochemical staining. The cells were cultured as follows: (1) Cultured in differentiation medium for 120 h (Untreated); (2) Cultured in differentiation medium for 96 h, and then additionally treated with DM and 10 µM Dex for 24 h (Dex); and (3) Cultured in differentiation medium for 96 h, and then additionally treated with DM, 10 µM Dex, and 20 µM Wedeloractone for 24 h (Mal) (size bar = 150 µm). Figure 3b shows the average muscle fiber diameter. Figure 3c shows the expression level of Atrogin-1 and Murf-1 at the mRNA level through qPCR analysis (*=p<0.05, **=p<0.01, and ***=p<0.001 mean statistical significance compared to the untreated group, and #=p<0.05, ##=p<0.01, and ###=p<0.001 mean statistical significance compared to the Dex-treated control group).

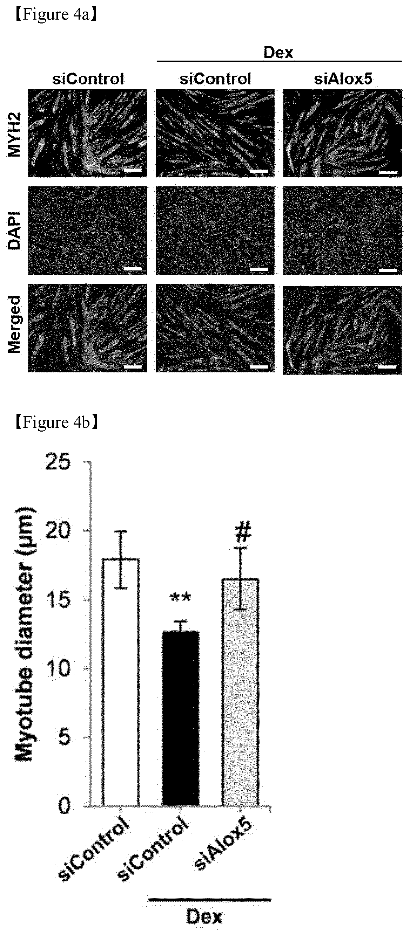

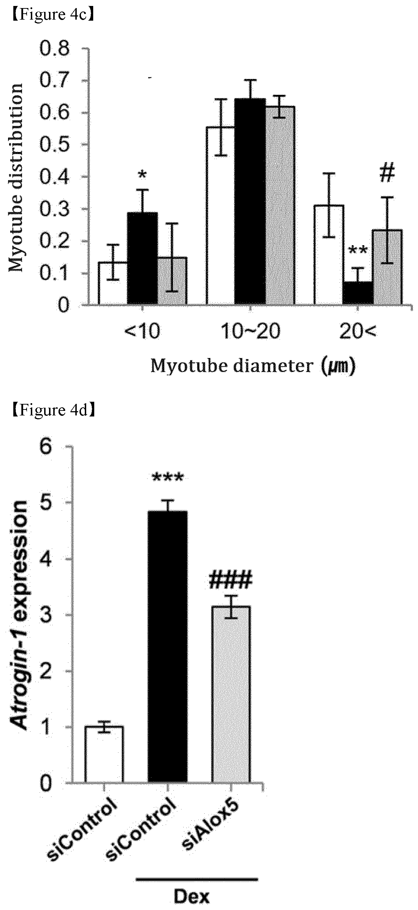

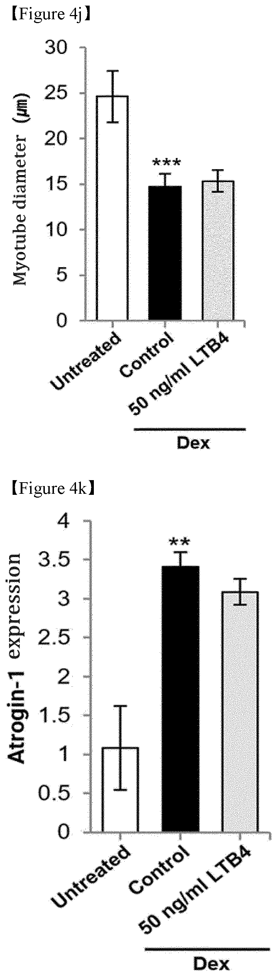

Figures 4a to 4d confirm the effect of inhibiting myotube atrophy by siAlox5. Figure 4a shows C2C12 muscle fibers stained with myosin heavy chain immunochemical staining. Figure 4b shows the average diameter of the muscle fibers. Figure 4c shows the distribution of muscle fiber diameter compared to the total number of muscle fibers. Figure 4d analyzes the expression level of Atrogin-1 at the mRNA level through qPCR analysis.

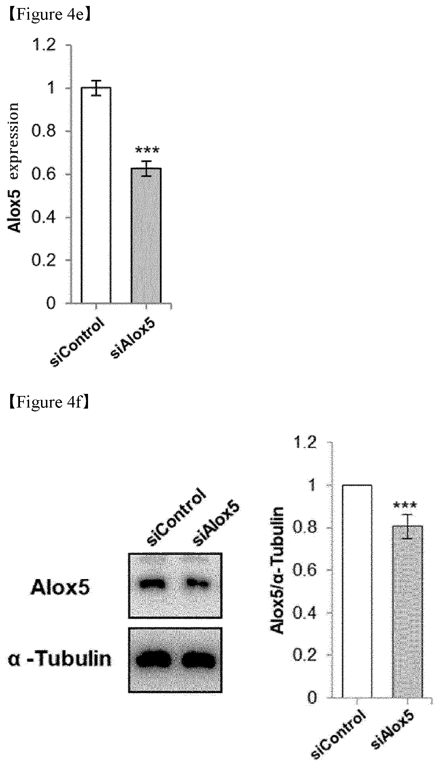

Figures 4e and 4f confirm the decrease in the expression level of Alox5 by siAlox5 through qPCR analysis and Western blot, respectively.

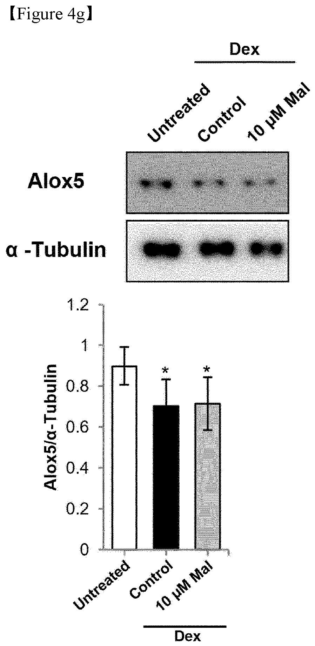

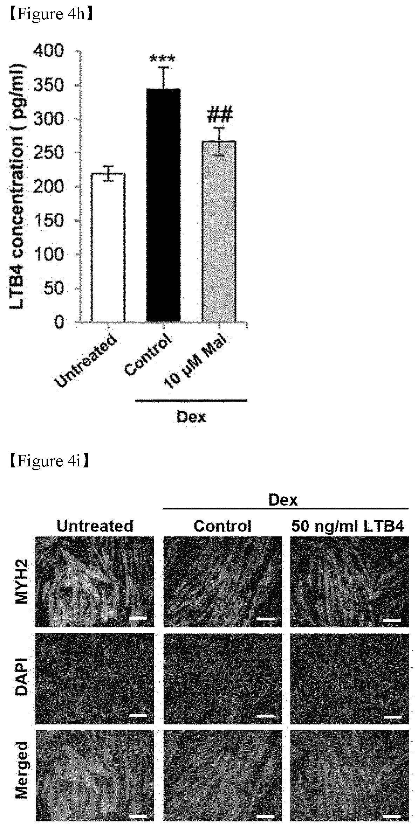

Figures 4g and 4h confirm the effect of malotilate treatment on Alox5 expression in Dex-treated myotubes. Figure 4g shows the expression of Alox5 through Western blot, and the expression level of the corresponding gene was quantified through the expression level of α-Tubulin. Figure 4h confirms the concentration of LTB4 accumulated in C2C12 muscle fibers through ELISA analysis.
Figures 4i to 4k confirm the effect of exogenous LTB4 treatment on Dex-treated myotubes. Figure 4i shows C2C12 muscle fibers stained with myosin heavy chain immunochemical staining. Figure 4j shows the average diameter of the muscle fibers. Figure 2k shows the expression level of Atrogin-1 at the mRNA level through qPCR analysis.

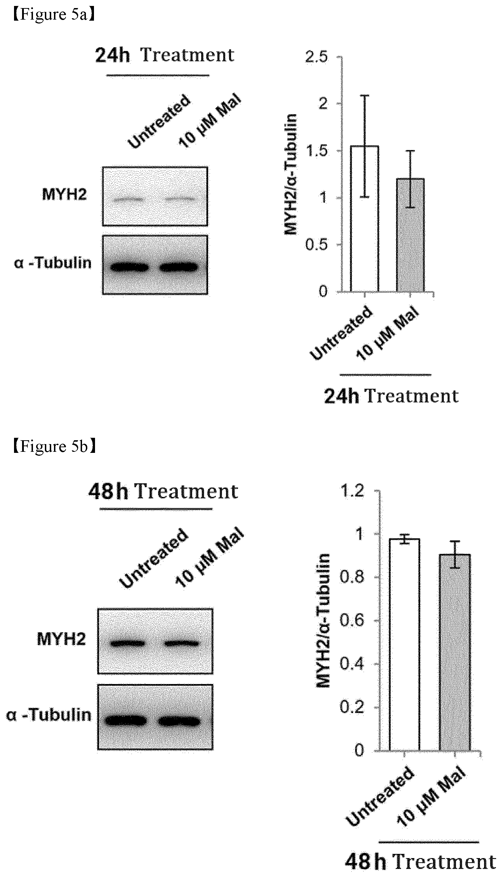

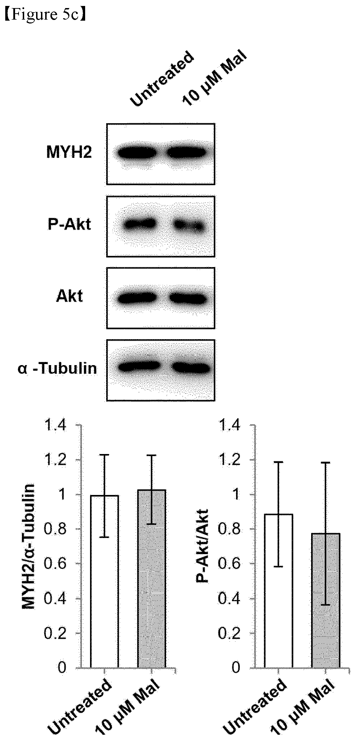

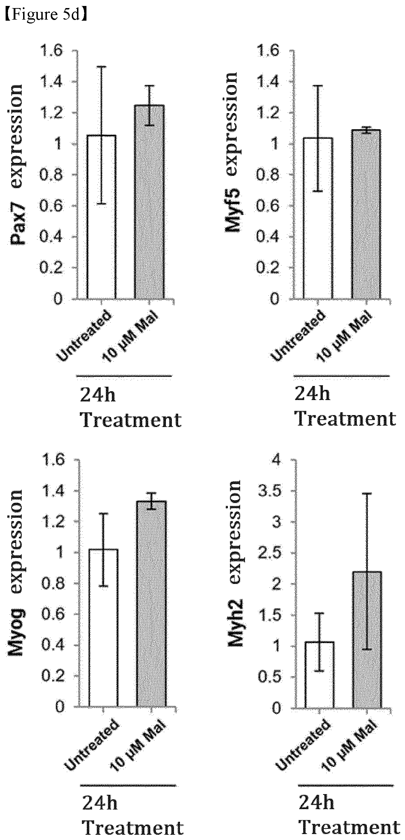

Figures 5a to 5d confirm the effect of malotilate treatment on myogenesis. Figures 5a and 5b confirm the expression of Myh2 through Western blot. Figure 5c confirms the level of Myh2 and phosphorylated Akt through Western blot. Figure 5d analyzes the expression levels of Pax7, Myf5, Myog, and Myh2 at the mRNA level through qPCR analysis (*=p<0.05, **=p<0.01, and ***=p<0.001 mean statistical significance compared to the untreated group, and #=p<0.05, ##=p<0.01, and ###=p<0.001 mean statistical significance compared to the Dex group).

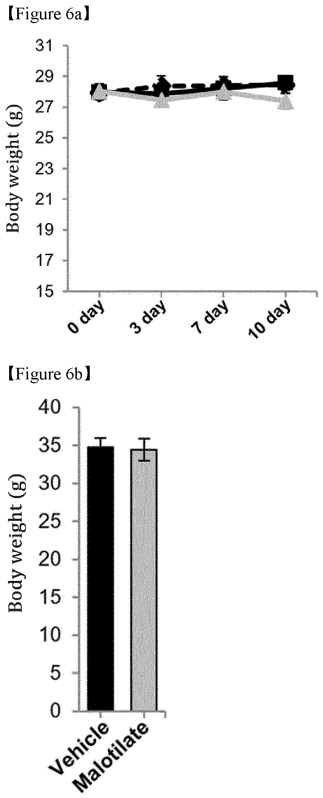

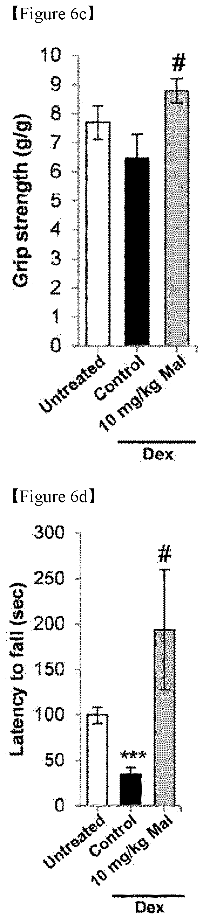

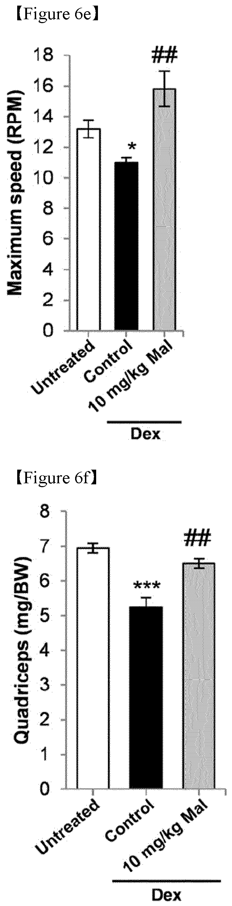

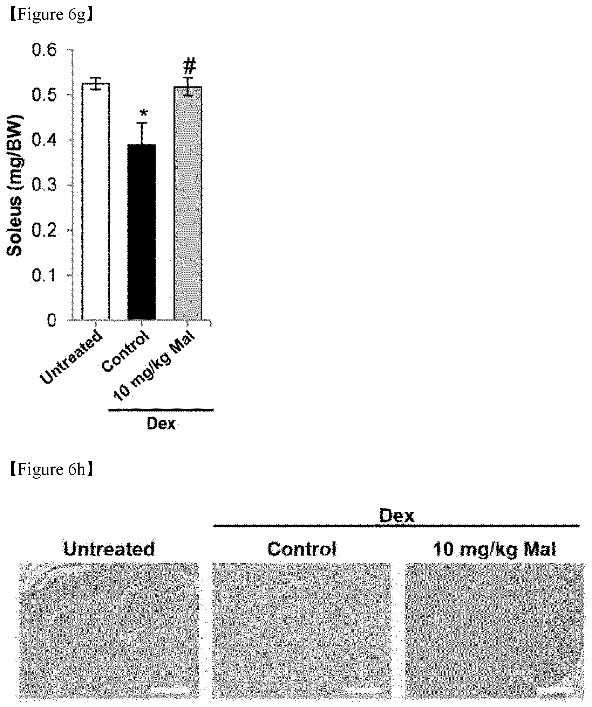

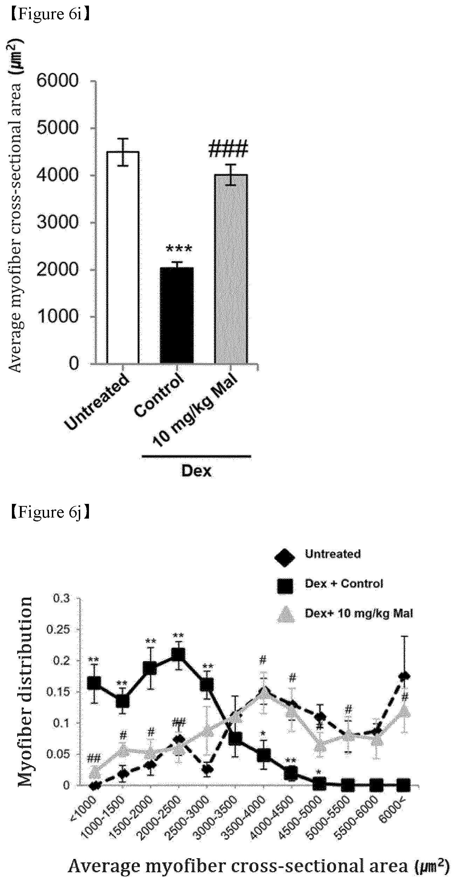

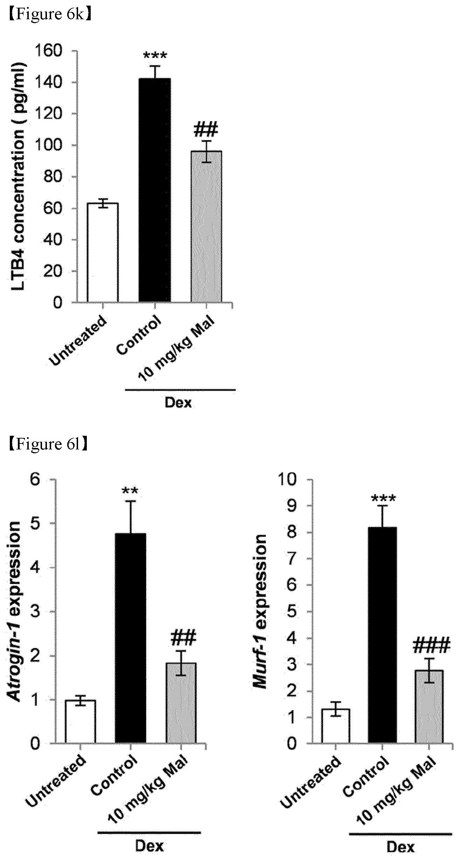

Figures 6a to 6l confirm the effect of inhibiting skeletal muscle atrophy due to malotilate treatment in the Dex-treated sarcopenia mouse model. Figure 6a shows the results of measuring the body weight of the mice. Figure 6b shows the results of measuring the body weight of the mice on the end day of drug treatment. Figure 6c shows the results of measuring the grip strength of the mice on the end day of drug treatment. Figure 6d shows the results of measuring the maximum exercise duration of the mice on the end day of drug treatment. Figure 6e shows the results of measuring the maximum sustainable exercise speed of the mice corresponding on the end day of drug treatment. Figure 6f shows the measured weight of the quadriceps muscle of the mice. Figure 6g shows the measured weight of the soleus muscle of the mouse. Figure 6h is a representative photograph showing the quadriceps muscle of a mouse after hematoxylin & eosin staining. Figure 6i shows the results of measuring the average large diameter of muscle fibers. Figure 6j shows the distribution of the large diameter size of muscle fibers compared to the total number of muscle fibers. Figure 6k shows the results of confirming the concentration of LTB4 accumulated in the quadriceps muscles of the mice through ELISA analysis. Figure 6l shows the expression levels of Atrogin-1 and Murf-1 in mouse quadriceps muscles at the mRNA level (*=p<0.05, **=p<0.01, and ***=p<0.001 mean statistical significance compared to the untreated group, and #=p<0.05, ##=p<0.01, and ###=p<0.001 mean statistical significance compared to the Dex group).

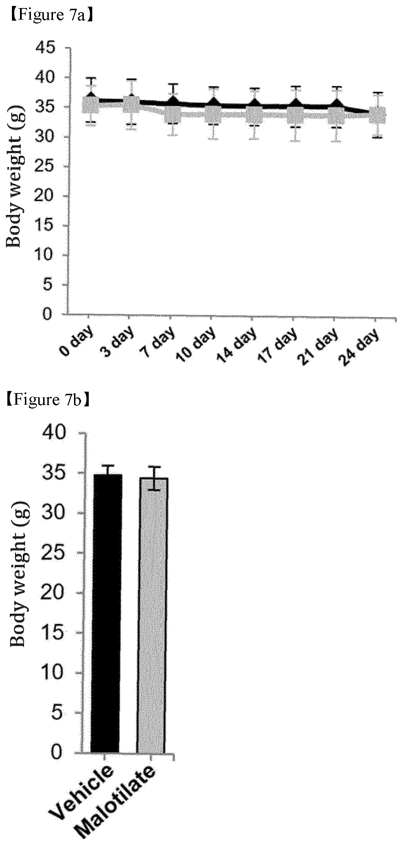

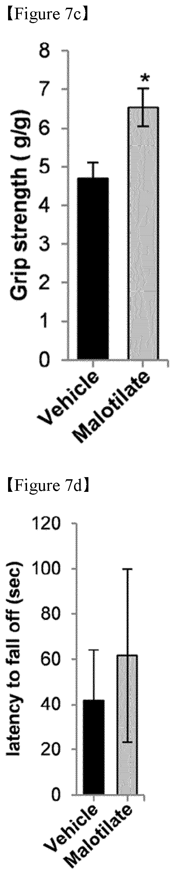

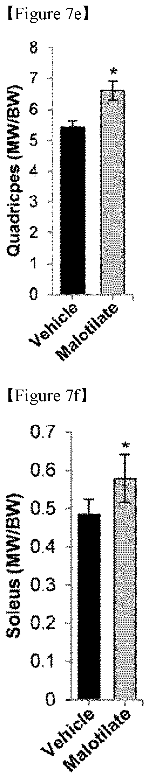

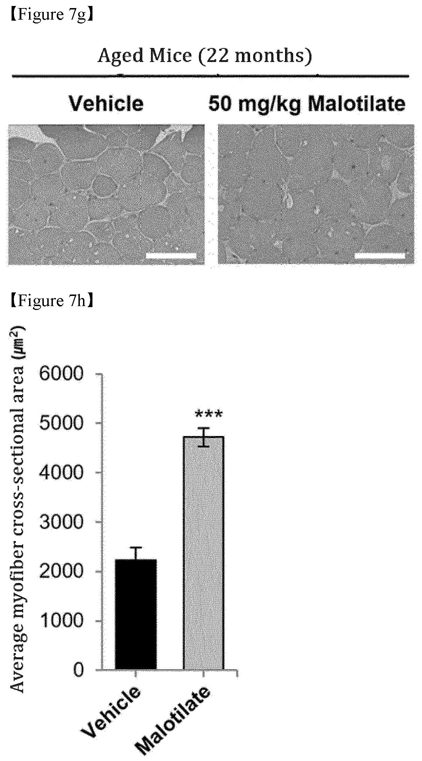

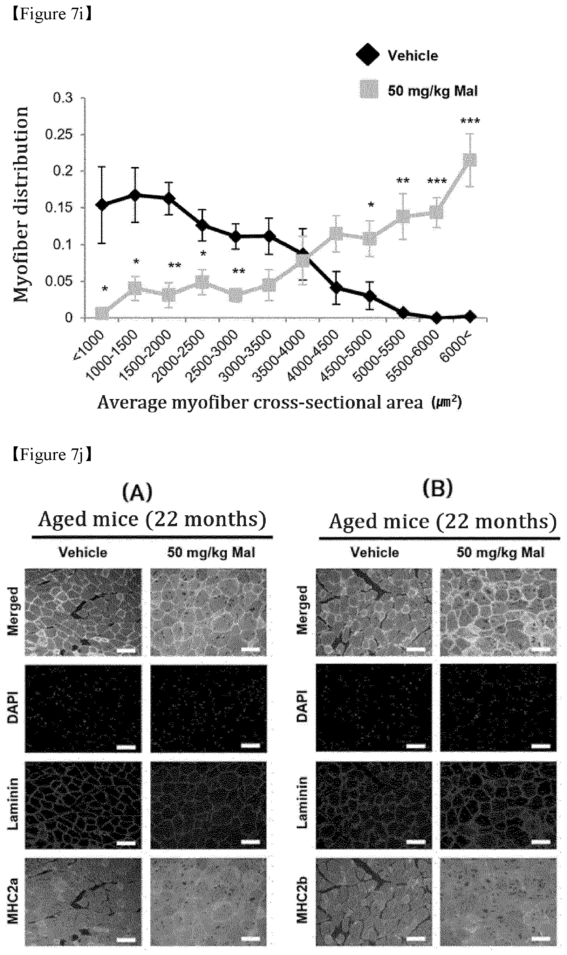

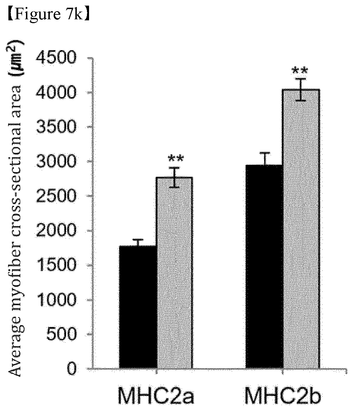

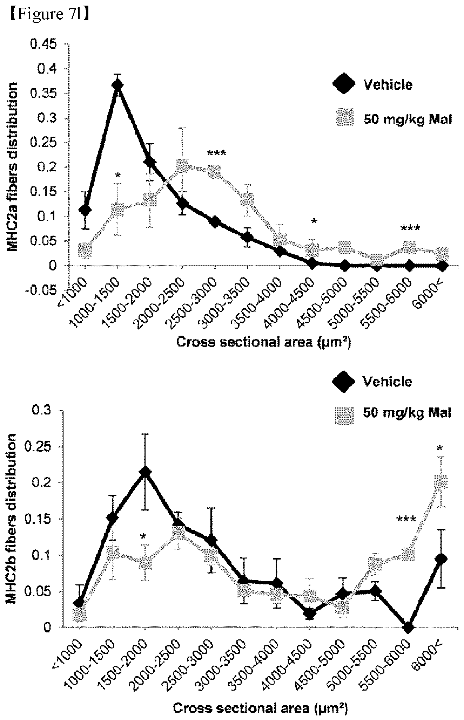

Figures 7a to 7l confirm the effect of inhibiting skeletal muscle atrophy due to malotilate treatment in a mouse model of sarcopenia due to aging. Figure 7a shows the results of measuring the body weight of mice during the 4-week experiment period. Figure 7b shows the results of measuring the body weight of the mice on the end day of drug treatment. Figure 7c shows the results of measuring the grip strength of the mice on the end day of drug treatment. Figure 7d shows the results of measuring the maximum exercise duration of the mice on the end day of drug treatment. Figure 7e shows the measured weight of the quadriceps muscle of the mice. Figure 5f shows the measured weight of the soleus muscle of the mice. Figure 7g is a representative photograph showing the quadriceps muscle of a mouse after hematoxylin & eosin staining. Figure 7h shows the results of measuring the average large diameter of muscle fibers. Figure 7i shows the distribution of the large diameter size of muscle fibers compared to the total number of muscle fibers. Figure 7j is a representative photograph shown after immunohistochemical staining with myosin heavy chain type 2A, 2B, and Laminin antibodies. Figure 7k shows the results of measuring the average large diameter of muscle fibers stained with myosin heavy chains 2A and 2B. Figure 7l shows the distribution of the large diameter of muscle fibers stained with myosin heavy chain 2A and 2B compared to the total number of muscle fibers (*=p<0.05, **=p<0.01, and ***=p<0.001 mean statistical significance compared to the untreated group, and #=p<0.05, ##=p<0.01, and ###=p<0.001 mean statistical significance compared to the Dex group).

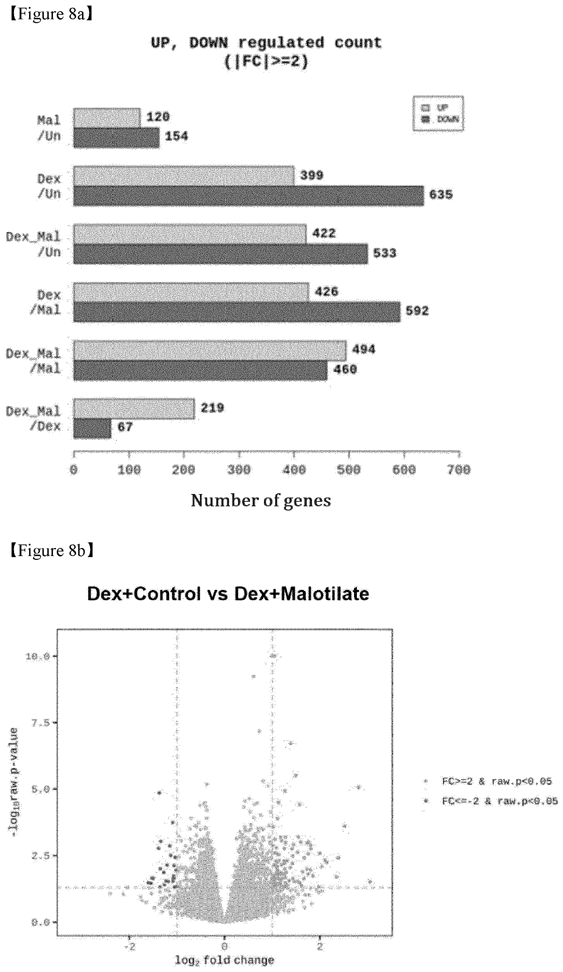

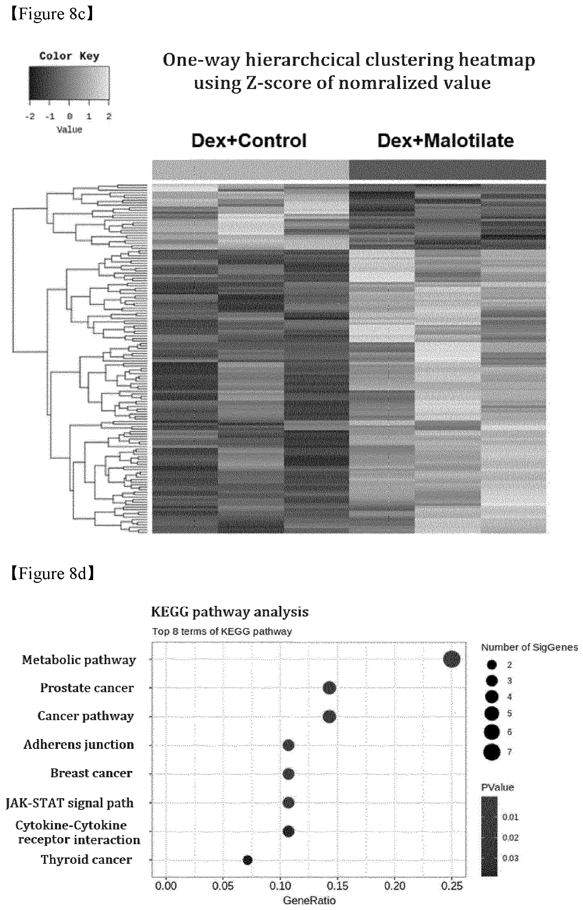

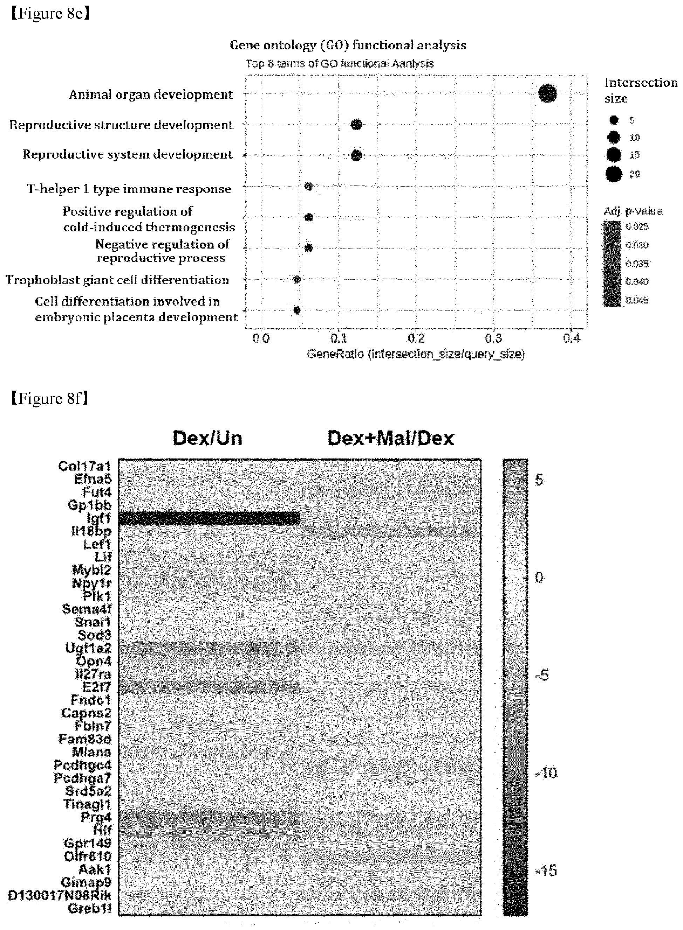

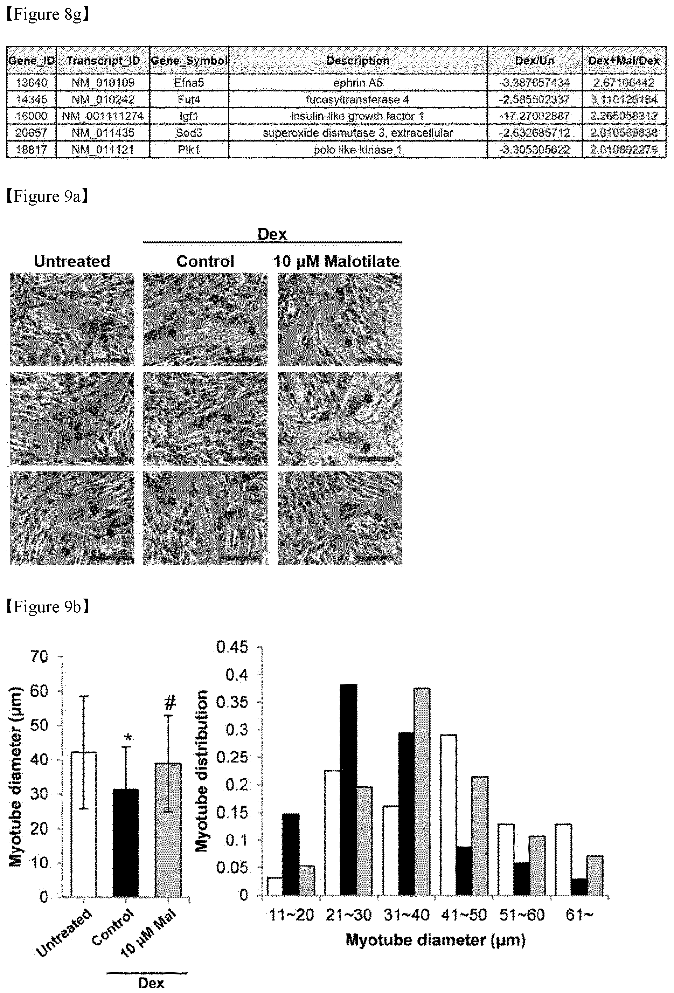

Figures 8a to 8g show analysis of the cell transcriptome obtained by treating myotubes suffering from atrophy due to Dex treatment with malotilate. Figure 8a shows genes up- or downregulated through intracellular transcriptome analysis of C2C12 muscle fibers. Figure 8b shows gene expression changes between Dex and Dex_Mal groups as a volcano plot. Figure 8c shows gene expression changes between Dex and Dex_Mal groups through a single hierarchical heatmap. Figure 8d shows gene expression changes between Dex and Dex_Mal groups with correlations between genes and functional roles. Figure 8e shows gene expression changes between Dex and Dex_Mal groups through KEGG (Kyoto Encyclopedia of Genes and Genomes) pathway analysis. Figure 8f is a heatmap quantitatively showing changes in expression levels of genes between Dex/Un and Dex_Mal/Dex. Figure 8g summarizes sarcopenia-related genes in the heatmap between Dex/Un and Dex_Mal/Dex.

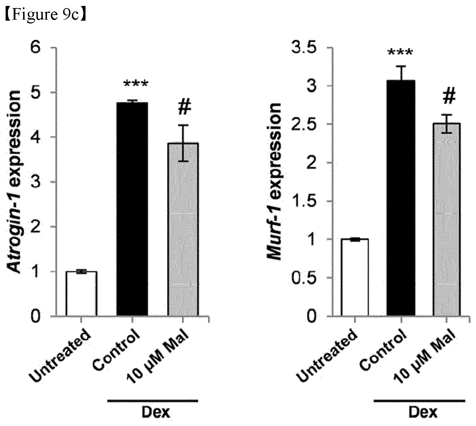

Figures 9a to 9c confirm the atrophy prevention effect of malotilate treatment in human skeletal muscle culture. Figure 9a shows muscle fibers of human-derived myoblasts stained with hematoxylin & eosin. Figure 9b shows the average muscle fiber diameter and distribution of muscle fibers. Figure 9c analyzes the expression levels of Atrogin-1 and Murf-1 at the mRNA level through qPCR analysis (*=p<0.05, **=p<0.01, and ***=p<0.001 mean statistical significance compared to the untreated group, and #=p<0.05, ##=p<0.01, and ###=p<0.001 mean statistical significance compared to Dex group).

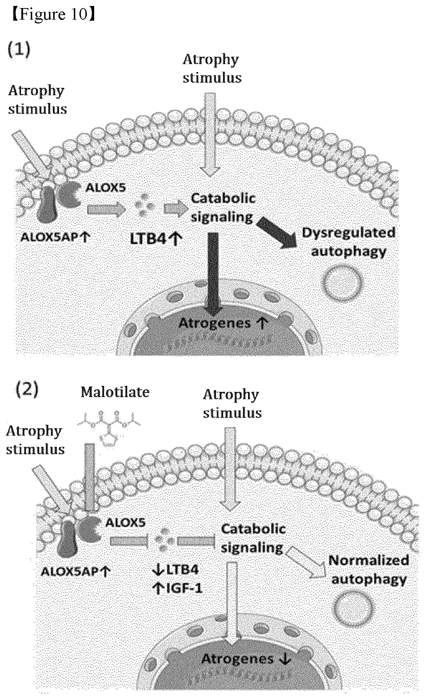

Figure 10 shows the relationship between Alox5 activity and sarcopenia and the mechanism of alleviating sarcopenia through malotilate.

### [Mode for Invention]

Hereinafter, the present invention will be described in detail.

The present invention relates to a pharmaceutical composition for preventing or treating sarcopenia comprising an Alox5 (Arachidonate 5-Lipoxygenase) inhibitor.

The Alox5 inhibitor may be a substance that inhibits the expression or activity of the Alox5 gene or protein.

The inhibitor may be a nucleic acid, antibody, aptamer, peptide, protein, compound, or natural product, and is not particularly limited as long as it inhibits the expression or activity of the Alox5 gene or protein.

For example, it may be a nucleic acid such as an antisense nucleotide, small interfering RNA (siRNA), short hairpin RNA (shRNA), micro RNA, or recombinant RNA that binds complementary to Alox5 mRNA. It may be an antibody, aptamer, peptide, protein, compound, or natural product that specifically binds to Alox5.

The Alox5 inhibitor may be selected from the group consisting of Malotilate, Wedelolactone, Zileuton, Nordihydroguaiaretic acid, Psoralidin, Docebenone, Licofelone, Lonapalene, Enazadrem, Cirsiliol, Picrinine, Atreleuton and mixtures thereof.

Preferably, the Alox5 inhibitor is malotilate.

The malotilate is represented by the following chemical formula (1).

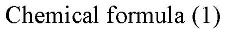

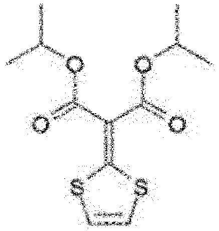

The prevention or treatment of sarcopenia may be achieved by reducing the production of leukotriene B4 (LTB4).

LTB4 is an active product of Alox5, and LTB4 increases in atrophic skeletal muscle. In the example of the present invention, it was confirmed that when myotubes atrophied by dexamethasone (Dex) treatment was treated with the composition of the present invention, the production of LTB4 was reduced, which had the effect of inhibiting myotube atrophy caused by Dex (Figure 4h). More specifically, LTB4 is involved in the dephosphorylation of FoxO3, a key regulator of the ubiquitin-proteasome pathway in skeletal muscle atrophy, through a catabolic signaling pathway. When LTB4 production is reduced, dephosphorylation of FoxO3 is inhibited, thereby preventing or preventing sarcopenia.

Additionally, skeletal muscle atrophy can be inhibited by reducing uncontrolled autophagy by reducing the production of LTB4. In the example of the present invention, it was confirmed that when Dex-treated myotubes are treated with an Alox5 inhibitor (e.g., malotilate), the ratio of LC3bII to LC3bI decreases and the expression of p62 increases, resulting in reducing unregulated autophagy (Figure 1g).

The present invention can prevent or treat sarcopenia by decreasing the expression of muscle atrophy regulators (atrogenes) or increasing the expression of anti-atrophy factors.

The muscle atrophy regulator is not limited thereto, but includes, for example, Atrogin-1 or MuRF-1.

The anti-atrophy factor is not limited thereto, but includes, for example, Efna5 (Ephrin A5), Fut4 (Fucosyltransferase 4), Igf1 (Insulin-like growth factor 1), Sod3 (Superoxide dismutase 3), or Plk 1 (Polo like kinase 1).

The sarcopenia may include sarcopenia due to any cause, such as aging, sepsis, starvation, chronic obstructive pulmonary disease, diabetes, or cancer, but preferably, the sarcopenia may be due to aging.

In the embodiment of the present invention, it was confirmed that skeletal muscle atrophy is inhibited through an increase in muscle mass and muscle fiber cross-sectional area by treatment with an Alox5 inhibitor (e.g., malotilate) in an animal model of sarcopenia due to Dex treatment or aging. As confirmed (Figures 6f to 6j and Figures 7e to 7i). Therefore, the composition of the present invention has excellent efficacy in preventing or treating sarcopenia.

The pharmaceutical composition of the present invention further comprises a pharmaceutically acceptable carrier, which is commonly used in preparation, such as lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate and mineral oil, but is not limited thereto. In addition to the above ingredients, the pharmaceutical composition of the present invention may further comprises lubricants, wetting agents, sweeteners, flavoring agents, emulsifiers, suspending agents, preservatives, etc. Suitable pharmaceutically acceptable carriers and formulations are described in detail in Remington's Pharmaceutical Sciences (19th ed., 1995). The appropriate dosage of the pharmaceutical composition of the present invention varies depending on factors such as formulation method, administration method, patient's age, weight, sex, severity of disease symptoms, food, administration time, administration route, excretion rate, and reaction sensitivity. Usually a skilled doctor can easily determine and prescribe an effective dosage for the desired treatment. Meanwhile, the dosage of the pharmaceutical composition of the present invention may be 0.01-2000 mg/kg (body weight) per day, but is not limited thereto.

The pharmaceutical composition of the present invention can be administered orally or non-orally, and when administered parenterally, it can be administered by intravenous injection, subcutaneous injection, intramuscular injection, intraperitoneal injection, transdermal administration, etc. It is preferable that the route of administration of the pharmaceutical composition of the present invention is determined depending on the type of disease to which it is applied.

The pharmaceutical composition of the present invention is prepared in unit dosage form by formulating with a pharmaceutically acceptable carrier and/or excipient according to a method that can be easily performed by a person skilled in the art to which the present invention pertains. Alternatively, it can be manufactured by placing it in a multi-dose container. At this time, the formulation may be in the form of a solution, suspension, or emulsion in an oil or aqueous medium, or may be in the form of an extract, powder, granule, tablet, or capsule, and may additionally comprise a dispersant or stabilizer.

The present invention relates to a functional health food for preventing or improving sarcopenia comprising an Alox5 (Arachidonate 5-Lipoxygenase) inhibitor.

The Alox5 inhibitor included in the functional health food for preventing or improving sarcopenia of the present invention, its mechanism of action, and the cause of sarcopenia are as described above.

The functional health food of the present invention can be manufactured and processed in the form of tablets, capsules, powders, granules, liquids, pills, etc. for the purpose of preventing or improving sarcopenia.

The functional health food of the present invention refers to food manufactured and processed using raw materials or ingredients with functional properties useful to the human body. It means taking it for the purpose of controlling nutrients for the structure and function of the human body or obtaining useful effects for health purposes such as physiological effects.

The functional health food of the present invention may comprise common food additives, and its suitability as a food additive is determined based on the specifications and standards in accordance with the general provisions of the food additive code and general test methods approved by the Food and Drug Administration, unless otherwise specified.

Examples of items listed in the Food Additives Code include chemical compounds such as ketones, glycine, calcium citrate, nicotinic acid, and cinnamic acid; natural additives such as persimmon color, licorice extract, crystalline cellulose, high-quality pigment, and guar gum; and mixed preparations such as L-glutamate sodium preparations, noodle added alkaline preparations, preservative preparations, and tar coloring preparations, but are not limited thereto.

For example, for functional health foods in the form of tablets, a mixture of the active agent with diluents, binders, disintegrants and other additives is granulated in a conventional manner, and then a lubricant is added thereto and is compressed for molding. Or, the mixture directly can be compression molded. In addition, the functional health food in the form of tablets may comprise flavoring agents, etc., if necessary.

Among capsule-type functional health foods, hard capsules can be manufactured by filling a regular hard capsule with a mixture of the active agent and additives such as diluents. Soft capsules can be manufactured by filling a mixture of the active agent with additives such as diluents into capsule material like gelatin. The soft capsule may further comprise plasticizers such as glycerin or sorbitol, colorants, preservatives, etc., if necessary.

The functional health food in the form of a pill can be prepared by molding a mixture of the above active agent and diluents, binders, disintegrants, etc., using a known method. If necessary, it can be coated with white sugar or another coating agent, or the surface can also be coated with a substance such as starch and talc.

Functional health foods in the form of granules can be prepared by mixing the active agent with diluents, binders, disintegrants, etc. into granules using a known method, and may further comprise flavoring agents, sweetners, etc., if necessary.

The functional health foods include beverages, meat, chocolate, foods, confectionery, pizza, ramen, other noodles, gum, candy, ice cream, alcoholic beverages, vitamin complexes, and health supplements.

The above functional health food can be applied orally as a nutritional supplement, and the form of application is not particularly limited. For example, when administered orally, the daily intake is preferably 5000 mg or less, more preferably 2000 mg or less, and most preferably 500 to 1500 mg or 650 mg per day. When formulated as a capsule or tablet, one tablet or capsule can be administered once a day with water.

Hereinafter, the present invention will be described in detail with reference to examples.

### Example. Confirmation of the effectiveness of preventing or treating skeletal muscle atrophy using Alox5 inhibitors

### Materials and methods

### 1. Reagents and antibodies

Dexamethasone (Dex) was purchased from Santa Cruz Biotechnology (TX, USA). Malotilate was purchased from Selleckchem (TX, USA). Leukotriene B4 (LTB4) was purchased from Tocris (Bristol, UK).

### 2. Cell culture

C2C12 murine skeletal muscle myoblasts were obtained from Koram Biotech (Republic of Korea). The cells were maintained in DMEM growth medium supplemented with 10% FBS, 1% penicillin, and streptomycin. When myoblasts proliferated to approximately 90%, they were differentiated into muscle fibers by treating them with DMEM differentiation medium supplemented with 2% HS, 1% penicillin, and streptomycin for 96 hours. Differentiated muscle fibers were treated with 10 µM Dex for 24 hours to induce sarcopenia. To evaluate the degree of differentiation of muscle cells, differentiating myoblasts were treated with the corresponding compound in differentiation medium for 24 or 48 hours.

### 3. Immunochemical staining

Muscle fibers were stained using immunochemical staining using myosin heavy chain antibody. Specifically, the muscle fibers were rinsed three times with PBS and then fixed with a 3.7% formaldehyde solution. After rinsing with PBS, a transparency process was performed with 0.2% TritonX-100 solution, and additional rinsing was performed with PBS. They were blocked with 1% bovine serum albumin solution (1% BSA in PBS-T) and incubated with primary antibody overnight at 4°C. After washing with PBS, the secondary antibody was treated for 1 hour. Afterwards, the myotubes were counterstained with 1 µM DAPI in PBS. Fluorescence images of stained myotubes in five different areas were obtained using a DMI 3000 B microscope (Leica, Germany) and analyzed using ImageJ 1.52 software (National Institutes of Health, Bethesda, MD, USA). Myotubes were classified as myosin heavy chain positive cells containing four or more nuclei, and the diameter of muscle fibers containing four or more nuclei was measured.

### 4. Western blotting

The concentration of intracellular protein was measured using the Bradford assay (Bio-Rad, USA, CA), and electrophoresis was performed by loading on 10% and 12% polyacrylamide gels. Afterwards, the separated proteins were transferred to a PVDF membrane, and the primary antibody used was diluted 1: 1000 in TBS-T containing 5% skim milk and incubated overnight at 4°C. Secondary antibodies were diluted 1:2000 and incubated for 35 minutes at room temperature (RT). Band intensity was measured using ImageJ 1.52 software (National Institutes of Health, USA), and intracellular protein was normalized to α-tubulin. The primary and secondary antibodies used are listed in Tables 1 and 2, respectively.

**[Table 1]**

| **Primary antibody** | **Clone** | **Company** | **Catalog No.** | **Dilution** |
|---|---|---|---|---|
| α-Tubulin | Polyclonal | Invitrogen | PA5-29444 | 1:5000 |
| Puromycin (12D10) | Monoclonal | Millipore | MABE-343 | 1:25000 |
| Atrogin-1/MAFbx (F-9) | Monoclonal | SANTA CRUZ | Sc-166806 | 1:100 |
| FoxO3a (D19A7) | Monoclonal | CST | #12829 | 1:1000 |
| Phospho-FoxO3a (Ser253) | Polyclonal | CST | #9466 | 1:1000 |
| Myosin heavy chain 2 (A4.7) | Monoclonal | SANTA CRUZ | Sc-53095 | 1:1000 |
| P62/SQSTM1 | Polyclonal | Merck | P0067 | 1:1000 |
| LC3B | Polyclonal | Merck | L7543 | 1:1000 |
| 5-Lipoxygenase | Monoclonal | Abcam | AB169755 | 1:1000 |
| Phospho-Akt (Ser473) | Polyclonal | CST | 49271 | 1:1000 |
| Akt | Polyclonal | CST | 49272 | 1:1000 |
| Laminin | Polyclonal | Abcam | AB11575 | 1:30 |
| Myosin heavy chain Type llA | Monoclonal | DSHB | SC-71 | 2-5 µg/ml |
| Myosin heavy chain Type llB | Monoclonal | DSHB | BF-F3 | 2-5 µg/ml |

**[Table 2]**

| **Secondary antibody** | **Conjugated use** | **Company** | **Catalog No.** | **Dilution** |
|---|---|---|---|---|
| Horse anti-Mouse IgG HRP | HRP | CST | #7074 | 1:2000 |
| Goat anti-Rabbit IgG HRP | HRP | CST | #7074 | 1:2000 |
| Alexa Fluor^{®} 488 Goat anti-mouse IgG (H+L) | Alexa Fluor 488 | INVITROGE N | A11001 | 1:1000 |
| Goat anti-Rabbit IgG h+l Dylight^{®} 594 Conjugated | Dylight^{®} 594 | Bethyl | A90-116D4 | 1:1000 |

### 5. RT-PCR and quantitative real-time PCR

For quantitative real-time PCR, transcript levels of genes of interest were analyzed using the StepOnePlus real-time PCR system (Applied Biosystems, UK). Total RNA was reverse transcribed to prepare cDNA using AccuPower^{®}RT PreMix (Bioneer Corporation). The obtained cDNA was subjected to real-time PCR according to the manufacturer's instructions with the following changes. PCR was performed in triplicate in a total volume of 20 µL 2X Power SYBR Green PCR Master Mix (Enzynomics, Republic of Korea) containing 200 nM (final concentration) of each specific primer and 1 µL of cDNA synthesized from 1 µg of total RNA. PCR amplification consisted of incubating the mixture at 95°C for 10 minutes, followed by 40 cycles of denaturation, annealing, and extension. Denaturation was performed at 95°C for 15 s, annealing was performed at 60°C for 1 min, and extension was performed at 72°C for 20 s with fluorescence detection at 72°C after each cycle. After the final cycle, melting point analysis of all samples was performed within the range of 60 to 95°C using continuous fluorescence detection. Specific cDNA samples were included in each run and used as reference material for comparison between runs. The expression level of GAPDH was used to calculate and normalize the expression levels of all other genes. Results were expressed as relative expression levels for each gene. The synthesized cDNA was amplified by quantitative real-time PCR (qRT-PCR) or standard PCR. The PCR primers used in this experiment are shown in Table 3 below.

**[Table 3]**

| **Primer name** | **Primer sequence** | **Size (bp)** | **Accession Number** |
|---|---|---|---|
| Gapdh | F: CTCCACTCACGGCAAATTCA | 120 | NM_001289726 |
| | R: GCCTCACCCCATTTGATGTT | | |
| Atrogin-1 | F: CAGAGAGCTGCTCCGTCTCA | 178 | NM_026346 |
| | R: ACGTATCCCCCGCAGTTTC | | |
| Murf-1 | F : CCGAGTGCAGACGATCATCTC | 198 | NM_001039048 |
| | R: TGGAGGATCAGAGCCTCGAT | | |
| FoxO3a | F: TGGAGTCCATCATCCGTAGTGA | 147 | NM_019740 |
| | R: CTGGTACCCAGCTTTGAGATGAG | | |
| Pax7 | F : CACAGAGGCAGAGCTGATTGC | 157 | NM_011039 |
| | R: CCAATTGAGGAGAGTGACAGGTT | | |
| Myf5 | F : AGCTGGGCAGAATACGTGCTT | 112 | NM_008656 |
| | R: AGAACAGGCAGAGGAGAATCCA | | |
| Myod1 | F : TGTCCTTTCGAAGCCGTTCT | 169 | NM_010866 |
| | R: TGCAGCCAGAGTGCAAGTG | | |
| Myogenin | F : AGCGCAGGCTCAAGAAAGTG | 181 | NM_031189 |
| | R: CCGCCTCTGTAGCGGAGAT | | |
| Myh2 | F : GATCACCACGAACCCATATGATT | 183 | NM_001039545 |
| | R: TTCATGTTCCCATAATGCATCAC | | |
| Alox5 | F: TTCCCATGTTACCGCTGGAT | 100 | NM_009662.2 |
| | R: GCTGCTTGAGGATGTGAATTTG | | |
| Gapdh | F: CTGCACCACCAACTGCTTAGC | 107 | NM_002046 |
| | R: TCTTCTGGGTGGCAGTGATG | | |
| Atrogin-1 | F: GGAACTACTCCAGACCCTCTACACA | 103 | NM_148177 |
| | R: CTCCATCCGATACACCCACAT | | |
| Murf-1 | F: TTGACTTTGGGACAGATGAGGAA | 102 | NM_032588 |
| | R: CCAGCTCCTTACTGGTGTCCTT | | |

### 6. LTB4 Enzyme-linked immunoprecipitation method

An LTB4 ELISA kit was purchased from MyBioSource (CA, USA). The concentrations of LTB4 in skeletal muscles and cell lysates were measured by the enzyme-linked immunoprecipitation method according to the manufacturer's instructions. Samples (100 µL) were maintained at room temperature (RT) for 1 hour in 96 wells with polyclonal LTB4 antibody. Afterwards, an enzymatic reaction was performed to stop the process, and the absorbance was measured at 405 nm.

### 7. Animal studies

Animal studies were performed under the auspices of the Institute for Laboratory Animal Research Guide for the Care and Use of Laboratory Animals. Additionally, the animal studies were approved by the Gwangju Institute of Science and Technology Animal Care and Use Committee (study approval number GIST-2021-059). 12-week-old male C57BL/6 mice were purchased from Damool Science (Daejun, Republic of Korea).

### 8. Dexamethasone-induced sarcopenia mouse model

12-week-old male C57BL/6J mice were divided into 3 groups of 5 each and treated with drugs as follows: 1) PBS with 5% DMSO and 5% Tween 80 (Vehicle group); 2) 15 mg/kg of Dexamethasone (at a concentration of 15 mg/kg, in PBS containing 5% DMSO and 5% Tween 80) (Dexamethasone group); and 3) 10 mg/kg of malotilate (at a concentration of 10 mg/kg, in PBS containing 15 mg/kg Dexamethasone, 5% DMSO, and 5% Tween 80) (Malotilate group). Those were injected intraperitoneally (solution volume = 200 µL) for 2 weeks. Afterwards, the muscle function and conditions of the mice were analyzed.

### 9. Sarcopenia model using aging mice

Male C57BL/6J mice over 21 months of age were divided into 2 groups of 5 and treated with drugs as follows: 1) 0.5% carboxyl methyl with 6.73% DMSO (Vehicle group) and 2) 50 mg/kg of malotilate (at a concentration of 50 mg/kg, 0.5% carboxyl methyl with 6.73% DMSO) (Malotilate group). Those were administered orally daily for 4 weeks. Afterwards, the function and condition of the muscles were analyzed.

### 10. Measurement of grip strength

Grip strength was measured with the BIO-GS3 grip strength test meter (Bioseb, FL, USA). Grip strength was measured by placing the mouse on a grid and gently pulling it back with all four paws until the mouse fell off the grid. The trials were conducted with a time interval of 30 seconds, and the largest value among the three trials was selected as the representative muscle strength.

### 11. Muscle fatigue test

Muscle fatigue was measured from two different models using Rotarod (Ugo Basile, Italy). One is a constant speed model that measures how long movement can be continued on a cylinder rotating at a constant speed, and the other model is an acceleration model that measures how fast the mouse can continue movement when the speed of the cylinder increases at regular intervals. Mice were trained to adapt from 5 rpm to 15 rpm one day before measurement in the above two models. During training, when the mouse failed to continue the exercise more than 4 times within 1 minute and fell, it was determined that the mouse could no longer continue the exercise, and the training was terminated.

### 12. Muscle dissection and histological analysis

Before sacrifice, mice were anesthetized by intraperitoneally administering 22 mg/kg Ketamine (Yuhan, Republic of Korea) and 10 mg/kg xylazine (Bayer, Republic of Korea) by IP injection, and then samples were collected. Quadriceps, gastrocnemius, tibialis anterior and soleus muscles were sampled and their weights were measured. The muscles sampled for immunohistostaining were cultured overnight at 4°C with 4% paraformaldehyde, fixed, embedded in paraffin solution, and stored at -80°C. Cutting and hematoxylin & eosin staining were carried out at the Animal Research Facility of the Gwangju Institute of Science and Technology, Republic of Korea. Hematoxylin & eosin staining was performed using the kit (Merck, Germany). The cross-sectional area of muscle fibers was measured using ImageJ 1.48 software (National Institutes of Health, USA).

### 13. Immunohistochemical staining

Immunohistochemical staining was performed using myosin heavy chain types 2A and 2B (DSHB, IA, USA) and anti-laminin antibody (Abeam, Cambirdge, UK). Counterstain was stained with 1 µM DAPI solution. The cross-sectional area of muscle fibers was visualized using LEICA DM 2500 (Leica, Germany) and then measured using ImageJ 1.48 software (National Institutes of Health, USA).

### 14. RNA sequencing

The concentration of total RNA was measured using Quant-IT RiboGreen (Invitrogen, #R11490). To evaluate the overall integrity of RNA, TapeStation RNA screentape (Agilent, #5067-5576) was performed. To prepare RNA of high purity, only RNA with a RIN value of 7.0 or higher was used for RNA library construction.

Only 0.5 µg of total RNA from each sample was used for library formation by the Illumina TruSeq Stranded Total RNA Library Prep Gold Kit (Illumina, Inc., San Diego, CA, USA, #20020599). The first step in the workflow is to remove rRNA from total RNA. Following this step, the remaining mRNA was fragmented into small pieces using divalent cations at elevated temperature. The cut RNA fragment was cloned into double-stranded cDNA using SuperScript II reverse transcriptase (Invitrogen, #18064014) and random primers DNA polymerase I, RNase H, and dUTP. This cDNA fragment then underwent a final repair process, addition of a single 'A' base, and adapter ligation, and the product was purified and enriched by PCR to create the final cDNA library.

Libraries were quantified using the KAPA Library Quantification Kit for Illumina Sequencing Platforms according to the qPCR Quantification Protocol Guide (KAPA BIOSYSTEMS, #KK4854) and qualified using TapeStation D1000 ScreenTape (Agilent Technologies, #5067-5582). The indexed libraries were then submitted to Illumina NovaSeq (Illumina, Inc., San Diego, CA, USA), and paired-end (2 × 100 bp) sequencing was performed at Macrogen Incorporated.

### 15. Human skeletal myoblast culture and experimentation

Human-derived myoblasts were purchased from Thermo-Fisher Scientific (USA). Myoblasts were thawed using a water bath, centrifuged at 180 g for 5 min at room temperature, and washed with 20 mL DM. Myoblasts were then resuspended in DM and seeded in a 12-well plate at a density of 4.8 × 10⁴ cells/well. After 48 hours, myoblasts were treated with compounds of interest for 24 hours. Myotubes were stained with H&E. After light microscopy analysis of DIC captured images (Olympus CKX41), myotubes were measured using ImageJ 1.48 software (National Institutes of Health, USA).

### 16. Statistical analysis

Statistical significance was determined using the Student's t-test. A p-value of less than 0.05 was considered as statistically significant. Unless otherwise mentioned, all data shown are representative of more than three experimental repeats and the graph error bars are standard deviation.

### Results

### 1. Alox5 inhibitors (malotilate, zileuton, and wedelolactone) inhibit atrophy in the myotubes of mice treated with glucocorticoids.

A glucocorticoid treatment model was used to investigate the effect of Alox5 inhibitors on myotube atrophy. Malotilate, zileuton, and wedelolactone were used as Alox5 inhibitors. This model was chosen because glucocorticoid levels increase due to aging and various types of skeletal muscle atrophy, such as sepsis, starvation, chronic obstructive pulmonary disease, diabetes, and cancer. Myotubes from mice treated with the Dex (glucocorticoid) showed decreased diameter and a greater proportion of thinner myotubes, indicating myocardial atrophy. Malotilate treatment inhibited the effect of Dex on myotube atrophy (Figures 1A to 1C). Atrogin-1 and MuRF-1 are important muscle atrophy regulators (atrogenes) that are upregulated in skeletal muscle wasting. Malotilate treatment inhibited the upregulation of Atrogin-1 and MuRF-1 in Dex-treated myotubes (Figure 1D). Moreover, total protein synthesis was reduced by Dex treatment and restored by malotilate treatment (Figure 1e). The transcription factor FoxO3a (forkhead box O3) is a key regulator of the ubiquitin-proteasome pathway in skeletal muscle atrophy. Dephosphorylation of FoxO3a by the catabolic signaling pathway induced translocation from the cytoplasm to the nucleus and upregulation of target muscle atrophy regulators. FoxO3a phosphorylation levels were decreased in myotubes treated with Dex and increased by malotilate treatment (Figure 1f), accompanied by a decrease in Atrogin-1 expression. Dysregulated autophagy is a key feature of skeletal muscle atrophy. Dex treatment increased autophagy in myotubes (increased ratio of LC3bII to LC3bI and decreased expression of p62) as previously described. Autophagy in Dex-treated myotubes was reduced by malotilate treatment (Figure 1g).

In addition, referring to Figures 2 and 3, it was confirmed that the diameter of C2C12 myotubes decreased when treated with dexamethasone, and as a result of treatment with Alox5 inhibitors, zileuton, wedelolactone, and malotilate, the diameter of muscle fibers was recovered. It was also confirmed that the expression levels of the UPS-related enzymes, Atrogin-1 and Murf-1, increased upon treatment with dexamethasone, but as a result of treatment with Alox5 inhibitors zileuton, wedelolactone, and malotilate, the expression levels of Atrogin-1 and Murf-1 decreased.

### 2. Alox5 siRNA gene knockdown prevents myotube atrophy associated with increased levels of leukotriene B4, an Alox5 product.

Malotilate has been characterized as a selective inhibitor of Alox5. The effect of Alox5 gene knockdown on myotube atrophy was evaluated in Dex-treated myotubes. Alox5 siRNA reduced gene expression and inhibited myotube atrophy in Dex-treated myotubes (Figures 4a-c). Gene knockdown was accompanied by a decrease in Atrogin-1 expression (Figure 4d). The ability of siRNA to reduce Alox5 expression was confirmed by qPCR and Western blotting (Figures 4e-f).

Treatment with malotilate, an Alox5 enzyme inhibitor, did not affect Alox5 expression in Dex-treated myotubes (Figure 4g). Leukotriene B4 (LTB4) is an Alox5 product involved in inflammatory responses and catabolic pathways such as NF-κ and FoxO signaling. LTB4 levels were found to be increased in Dex-treated myotubes and lowered by malotilate (Figure 4h). Accordingly, application of exogenous LTB4 did not affect Dex-treated myotubes or the expression of Atrogin-1 (Figures 4i-k).

### 3. Malotilate does not have a significant effect on the myogenic program.

To evaluate the effect of malotilate treatment on myogenesis, mouse myotubes were induced to differentiate in the presence of malotilate. Expression of the differentiation marker myosin heavy chain type 2 (Myh2) was not affected by malotilate treatment during myogenesis (Figures 5a-b). Increased phosphorylation of Akt is a key signaling event during myogenesis, but the phosphorylation level of Akt in differentiated myotubes was not significantly altered by malotilate treatment (Figure 5c). Expression analysis of master gene regulators of the myogenic program (Pax7, Myf5, MyoG, and Myh2) showed no significant changes after 24 h of malotilate treatment (Figure 5d).

### 4. Malotilate inhibits skeletal muscle atrophy in a glucocorticoid treatment model.

The Dex glucocorticoid treatment model was chosen as the first test to investigate the effectiveness of malotilate to prevent skeletal muscle atrophy *in vivo.* Malotilate treatment for 14 days had no significant effect on the body weight of Dex-treated mice (Figures 6a-b). Malotilate treatment significantly increased grip strength in Dex-treated mice (Figure 6c). Dex treatment reduced latency to fall and maximum velocity in the rotarod performance test, both of which were significantly increased by malotilate treatment (Figures 6d-e). Assessment of skeletal muscle mass showed that malotilate treatment increased mass in the quadriceps and soleus muscles of dexamethasone-treated mice (Figures 6f-g). The average muscle fiber cross sectional area (CSA) and the proportion of fibers with larger CSA were increased by malotilate treatment (Figures 6h-j). ELISA analysis indicated that the level of the Alox5 product, LTB4, is increased in Dex-treated muscle and lowered by malotilate treatment (Figure 6k). Malotilate treatment also reduced the expression of muscle atrophy regulators atrogin-1 and MuRF-1 in Dex-treated mice (Figure 6l).

### 5. Malotilate inhibits skeletal muscle atrophy in aged mice.

To evaluate the potential of malotilate to treat age-related skeletal muscle atrophy (termed sarcopenia), aged mice were treated by oral delivery for 4 weeks. Malotilate treatment had no effect on body mass of aged mice (Figures 7a-b). Grip strength was significantly increased by malotilate treatment (Figure 7c). The mean latency to fall increased in malotilate-treated mice but did not reach statistical significance (Figure 7d). Quadriceps and soleus muscle mass significantly increased in malotilate-treated aged mice (Figures 7e-f). Histological analysis of the quadriceps muscle showed that the average muscle fiber cross-sectional area was increased by malotilate treatment (Figure 7h). The myofiber diameter distribution indicates that large myofibers (>5000 µm²) represent a much larger proportion in malotilate-treated aged mice (Figure 7i). Immunohistological analysis of fast twitch fiber types IIA and IIB, which undergo preferential atrophy during aging compared to slow class I twitch fibers, indicates that malotilate treatment preserves the CSA of fast twitch fibers (Figures 7j-l).

### 6. Cellular transcriptomic analysis of malotilate-treated myotubes

To obtain an overview of the mechanism by which Alox5 inhibition by malotilate inhibits muscle atrophy, myotubes undergoing Dex-induced atrophy were treated with malotilate and their cellular transcriptomes were examined using RNA Seq. Expression patterns were compared among untreated, Dex-treated myotubes; normal myotubes; or myotubes treated with malotilate alone (Figure 8a). Heatmaps and volcano plots showed a total of 139 differentially expressed genes (DEGs) between myotubes treated with Dex or Dex plus malotilate (Figures 8b-c). Kyoto Encyclopedia of Genes and Genomes (KEGG) pathway analysis showed that metabolic pathways were highly upregulated by malotilate treatment (Figure 8d). Gene Ontology (GO) functional analysis indicates that organ development is the best biological process increased in myotubes treated with Dex plus malotilate compared to Dex alone (Figure 8e). A heatmap for genes showing differential expression in Dex plus malotilate and Dex alone compared to malotilate and untreated group is shown in Figure 8f. The expression of many genes known to be associated with skeletal muscle atrophy was regulated by malotilate treatment. Genes known to be associated with skeletal muscle atrophy are shown in Figure 8g.

### 7. Malotilate prevents atrophy in human skeletal muscle cultures.

Skeletal muscle progenitor cells from human donors were differentiated into myotubes and induced to undergo atrophy by dexamethasone treatment. Histological analysis indicates that co-treatment with malotilate can prevent atrophy of human myotubes, as indicated by increased mean myotube diameter and a greater proportion of mytotubes of larger size (Figures 9a-b). Malotilate treatment also significantly reduced the expression of muscle atrophy regulators, atrogin-1 and MuRF-1, in human myotubes (Figure 9c).

## Claims

1. A pharmaceutical composition for preventing or treating sarcopenia, comprising an Alox5 (Arachidonate 5-Lipoxygenase) inhibitor.

2. The pharmaceutical composition for preventing or treating sarcopenia according to claim 1, wherein the Alox5 inhibitor is a nucleic acid, antibody, aptamer, peptide, protein, compound or natural product.

3. The pharmaceutical composition for preventing or treating sarcopenia according to claim 1, wherein the Alox5 inhibitor is selected from the group consisting of Malotilate, Wedelolactone, Zileuton, Nordihydroguaiaretic acid, Psoralidin, Docebenone, Licofelone, Lonapalene, Enazadrem, Cirsiliol, Picrinine, Atreleuton and mixtures thereof.

4. The pharmaceutical composition for preventing or treating sarcopenia according to claim 1, wherein the prevention or treatment of sarcopenia is achieved by reducing the production of leukotriene B4 (LTB4).

5. The pharmaceutical composition for preventing or treating sarcopenia according to claim 1, wherein the prevention or treatment of sarcopenia is due to reduced expression of Atrogin-1 or MuRF-1; or increased expression of one or more genes selected from Efna5 (Ephrin A5), Fut4 (Fucosyltransferase 4), Igf1 (Insulin-like growth factor 1), Sod3 (Superoxide dismutase 3), and Plk 1 (Polo like kinase1).

6. The pharmaceutical composition for preventing or treating sarcopenia according to claim 1, wherein the sarcopenia is caused by aging.

7. A functional health food for preventing or improving sarcopenia, comprising an Alox5 (Arachidonate 5-Lipoxygenase) inhibitor.

8. . The functional health food for preventing or improving sarcopenia according to claim 7, wherein the Alox5 inhibitor is a nucleic acid, antibody, aptamer, peptide, protein, compound or natural product.

9. The functional health food for preventing or improving sarcopenia according to claim 7, wherein the Alox5 inhibitor is selected from the group consisting of Malotilate, Wedelolactone, Zileuton, Nordihydroguaiaretic acid, Psoralidin, Docebenone, Licofelone, Lonapalene, Enazadrem, Cirsiliol, Picrinine, Atreleuton and mixtures thereof.

10. The functional health food for preventing or improving sarcopenia according to claim 7, wherein the prevention or treatment of sarcopenia is achieved by reducing the production of leukotriene B4 (LTB4).

11. The functional health food for preventing or improving sarcopenia according to claim 7, wherein the prevention or treatment of sarcopenia is due to reduced expression of Atrogin-1 or MuRF-1; or increased expression of one or more genes selected from Efna5 (Ephrin A5), Fut4 (Fucosyltransferase 4), Igf1 (Insulin-like growth factor 1), Sod3 (Superoxide dismutase 3), and Plk 1 (Polo like kinase1).

12. The functional health food for preventing or improving sarcopenia according to claim 7, wherein the sarcopenia is caused by aging.
